# EUROPEAN PATENT APPLICATION

(11) **EP 2 053 131 A1**
(43) Date of publication of application: **29.04.2009**
(21) Application number: 07020527.3
(22) Date of filing: 19.10.2007
(51) Int. Cl.: C12Q 1/68

(54) **Method for determining methylation at deoxycytosine residues**

(71) Applicant: Ludwig-Maximilians-Universität München, 80359 München (DE)
(72) Inventor: The designation of the inventor has not yet been filed
(74) Representative: Weiss, Wolfgang

(57) **Abstract**

The present invention refers to a method and reagent kits for determining methylation at cytosine (dC) residues in nucleic acids, such as DNA.

## Description

The present invention refers to a method and reagent kits for determining methylation at cytosine (dC) residues in nucleic acids, such as DNA.

The analysis of DNA methylation patterns in epigenetics has become an important tool in biomedical diagnostics. The differential methylation of certain promoters has been correlated with various cancer types. In addition to the need for analyzing DNA methylation patterns, the development of specific inhibitors of methyltransferases, especially human Dnmt1, has become of great importance in the quest for novel antitumor agents. Inhibition of methyltransferases has been shown to reactivate tumor-suppressor genes that were silenced by promoter hypermethylation. To this date, the only reliable method to quantify methyltransferase activity is the incorporation of a radioactive label via tritiated S-adenosyl-methionine.

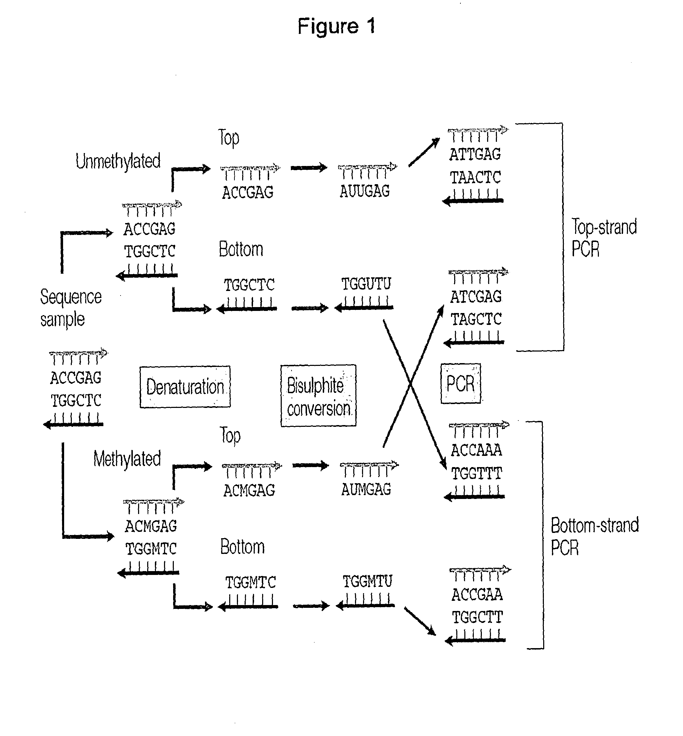

Currently, numerous assays for d(CpG) methylation based on bisulfite-treatment are available [see [1] for review]. Here, a sample comprising the gene of interest is split into two fractions. The first fraction is immediately sequenced; the second fraction is treated with bisulfite. Bisulfite preferentially reacts with non-methylated cytosine (dC) residues but not with 5-methylcytosine (^{5Me}dC) residues. Reaction with non-methylated dC causes rapid deamination of the base to form uracil (dU). The treated gene therefore contains nucleotides incorporating adenine (dA), guanine (dG), 5-methylcytosine (^{5Me}dC) and uracil (dU, instead of dC). Subsequent PCR of the bisulfite treated DNA converts the dU residues into thymidine (dT) residues, producing an amplicon with the nucleotides dG, dA, ^{5Me}dC and dT. This amplicon is finally sequenced and the sequence is compared with the sequencing data obtained from the untreated gene fragment. At all positions where the information dC was changed into dT, an unmodified dC was present in the original gene. A reaction scheme is shown in Fig. 1. All unchanged positions contain a ^{5Me}dC.

The major downside of the method is that the bisulfite treatment strongly damages and degrades the DNA. The data are hence frequently not reliable. Another downside is that the bisulfite reaction is performed directly on double-stranded DNA. However, some dC residues do not react under these conditions. Such incomplete reactivity, on the other hand, provides a serious disadvantage since the ^{5Me}dC information is captured only indirectly by determination of reacted dC residues.

Thus, a more efficient and reliable sequencing of the epigenetic information (positions of 5-methylcytosine) requires reagents which allow to react more selectively with either non-methylated cytosine or 5-methylcytosine.

A number of other reagents is known, which react selectively with either non-methylated cytosine or 5-methylcytosine. Hydrazine for example has been shown to react selectively with non-methylated cytosine, but it has to be used in pure form being volatile, toxic and potentially explosive. In combination with a piperidine cleavage of the hydrazine-reacted strands, this protocol has been extensively used for the sequencing of DNA [2] before the advent of the *Sanger* dideoxy method. OsO₄ and KMnO₄ have been shown to react selectively with 5-methylcytosine, but they also react to a large extent with thymidine bases, thus strongly limiting the practical value of this method. Hydroxylamine has been reported to function as a substitute for hydrazine in the classical *Maxam-Gilbert* sequencing method [3].

Further, it has been described that hydroxylamine reacts with non-methylated cytosine bases, but not with 5-methylcytosine bases [4a; 4b]. However, there has been no suggestion to use this method for genomic analysis and sequencing purposes. In particular, the prior art does not suggest the use of hydroxylamine for determining the methylation pattern of genes.

According to the present invention, a new chemistry has been developed which allows the selective reaction of non-methylated cytosine residues or 5-methylcytosine residues in order to obtain modified or damaged DNA-bases.

A first aspect of the present invention refers to a method for determining methylation at cytosine residues in DNA, comprising the steps:
(a) providing a sample comprising a single-stranded DNA segment comprising at least one cytosine residue,
(b) treating a fraction of the sample from step (a) with a first reagent which selectively reacts with 5-methylcytosine residues in said single-stranded DNA segment to obtain modified residues,
(c) treating a fraction of the sample from step (a) with a second reagent which selectively reacts with non-methylated cytosine residues in said single-stranded DNA segment to obtain modified residues, and
(d) detecting said modified residues.

A further aspect of the invention refers to a method for determining 5-methylcytosine residues in DNA, comprising the steps:
(a) providing a sample comprising a single-stranded DNA segment comprising at least one cytosine residue,
(b) treating a fraction of the sample from step (a) with a reagent which selectively reacts with 5-methylcytosine residues in said single-stranded DNA segment to obtain modified residues, and
(c) detecting said modified residues.

Still a further aspect of the present invention refers to a method for determining non-methylated cytosine residues in DNA, comprising the steps:
(a) providing a sample comprising a single-stranded DNA segment comprising at least one cytosine residue,
(b) treating a fraction of the sample from step (a) with a reagent which selectively reacts with non-methylated cytosine residues in said single-stranded DNA segment to obtain modified residues, and
(c) detecting said modified residues.

Still a further aspect of the present invention refers to a reagent kit for determining methylation at cytosine residues in DNA, comprising:
(a) a first reagent which selectively reacts with 5-methylcytosine residues in a single-stranded DNA segment to obtain modified residues,
(b) a second reagent which selectively reacts with non-methylated cytosine residues in a single-stranded DNA segment to obtain modified residues, and
(c) optionally further reagents for introducing a chemical DNA strand cleavage at modified residues and/or reagents for carrying out a DNA polymerase reaction.

The method of the present invention is carried out on a sample comprising a single-stranded DNA segment with at least one cytosine residue. The single-stranded DNA segment is preferably a genomic DNA segment from an eukaryotic organism, e.g. from an animal, particularly a human, or from plant material. The DNA sample may be obtained by standard methods known in the art.

Preferably the single-stranded DNA segment has a length of from about 2-20 nucleotides, more preferably from about 2-10 nucleotides, and is flanked by at least one non-single-stranded DNA fragment, e.g. a double- or triple-stranded DNA segment. More preferably, the single-stranded DNA segment is flanked on both sides by non-single-stranded segments. For example hybridization probes, e.g. oligonucleotides surrounding the target site of interest may be hybridized to the DNA, creating a short region of, for example 2-10, single-stranded bases containing one or multiple cytosine residues in question, for example in a d(CpG) sequence. If not stated otherwise, the term "cytosine residue", as used herein, comprises both non-methylated cytosine (dC) residues and 5-methylcytosine (^{5Me}dC) residues.

The cytosine residue to be analyzed is positioned in a single stranded region, while the flanking regions are double stranded. The reagents of the inventive method were found to react selectively with single stranded regions, while double stranded parts of the DNA are protected. Nucleobases in double strands do not react. In this way the positioning of the flanking primers provides a first level of site-selectivity. RNA strands or DNA strands or other oligomeric nucleic acids including mixtures can be used to create double or even triple strand regions flanking the single-stranded DNA segment to be analyzed. The single-stranded segment between the flanking duplex or triplex strands can vary in length and may contain one or multiple cytosine or 5-methylcytosine residues to be analyzed. Tagged, e.g biotinylated hybridization probes capable of hybridizing to the strand regions flanking the single-stranded DNA segment to be analyzed may be used for further enrichment/purification.

The DNA sample as prepared above may be split into two or more fractions. One fraction may be treated with a reagent that selectively reacts with 5-methylcytosine residues in the single-stranded DNA segment. The other fraction may be treated with a reagent selectively reacting with non-methylated cytosine residues in the single-stranded DNA segment. Further fractions may be taken e.g. as controls.

The selective reaction with 5-methylcytosine residues preferably comprises reacting said 5-methylcytosine residues with an electrophilic/oxidizing species under conditions wherein non-methylated cytosine residues are substantially inert. Further, the reaction is preferably carried out under conditions wherein thymidine residues are also substantially inert. The selectivity of the reaction is based on the lower oxidential potential of cytosine and thymidine as compared to 5-methylcytosine, as a result of which 5-methylcytosine is easier to oxidize. In this context, the electrophilic species selectively reacts with the C5-C6 double bond of 5-methylcytosine, causing oxidation of the aromatic ring to a non-aromatic system exhibiting a saturated C5-C6 bond.

The selective modification of 5-methylcytosine residues may be carried out by either reacting the single-stranded DNA segment directly with an electrophilic/oxidizing species or reacting said single-stranded DNA segment with an electrophilic/oxidizing species generated by oxidizing a precursor with an oxidant. In a preferred embodiment, the electrophilic species is generated, e.g. in situ, by oxidizing a precursor with an oxidant. Bromonium ions (Br⁺) generated from a suitable precursor (e.g. either Br₂ or Br) through mild oxidation are especially preferred as electrophilic species. Suitable sources of Br especially comprise bromide salts such as LiBr, NaBr or KBr. As oxidant, iodine-containing compounds such as ortho-iodoxybenzoic acid (IBX), Dess-Martin periodinane (DMP) and periodates, permanganates, perruthenates, vanadium (V)-containing compounds such as V₂O₅, molybdenum (VI)-containing compounds such as Na₂MoO₄, tungsten (VI)-containing compounds such as Na₂WO₄, or peroxides such as meta chloroperbenzoic acid are preferred. Instead of Br⁺, other electrophilic species such as NO⁺, NO₂⁺, SO₃H⁺, Cl⁺, I⁺, carbon based electrophils such as those used in Friedel Crafts acylations or alkylations, or electrophilic oxygen species such as those generated from the above mentioned peroxides may be used. Further, the reaction may comprise an agent capable of unfolding DNA structures present in longer DNA segments. Such agents comprise, but are not limited to, formamide, N,N-dimethylformamide, urea, guanidine hydrochloride, guanidinium isothiocyanate, imidazole or NaOH/EDTA. Preferably, the DNA-unfolding agent is used in a concentration of about 10 to 100 mM, e.g. about 50 mM. Treatment of the DNA with such reagents, in the preferred case with Br⁻ salts such as LiBr and an oxidant such as IO₄⁻ or V₂O₅, gives selective reaction at 5-methylcytosine sites.

The selective reaction of non-methylated cytosine residues preferably comprises reacting said non-methylated cytosine residues with a nucleophilic reagent under conditions wherein 5-methylated cytosine residues are substantially inert, with positions C4 and/or C6 of the non-methylated cytosine residues being preferred for an attack of the nucleophilic reagent. As a nucleophilic reagent, any reagent capable of nucleophilic attack at positions C4 and/or C6 of the non-methylated cytosine residues may be used. Preferred nucleophilic reagents comprise hydroxylamines and hydrazine species, with hydroxylamine, an N- or O-substituted hydroxylamine such as an oxime, or a substituted hydrazine being especially preferred. Neither 5-methylcytosine residues nor thymidine residues react under these conditions.

In a first embodiment, the detection comprises a chemical treatment, e.g. with a base such as piperidine, whereby a DNA strand cleavage occurs at positions of modified residues. The cleavage may be determined by known methods, e.g. by gel electrophoresis. In a further embodiment, the detection comprises carrying out a DNA polymerase reaction under conditions whereby blocking and/or misreading of the DNA polymerase reaction occurs at positions of modified residues. Thus, the presence or absence of methylation at cytosine residues may be determined by analyzing the reaction products of a polymerase reaction which may be carried out using standard or hot-start conditions, with hot-start conditions being preferred.

The polymerase reaction may be carried out under conditions where blocking at reacted dC residues occurs. In this embodiment, the polymerase is preferably a high-fidelity polymerase, e.g. Taq DNA polymerase, Pfu DNA polymerase, Vent DNA polymerase, Vent exo- DNA polymerase, KOD DNA polymerase and BstPoll DNA polymerase, or a high-fidelity mutant of a high-fidelity polymerase.

In an alternative embodiment, the polymerase reaction is carried out under conditions where misreading at reacted dC residues occurs. In this embodiment the polymerase reaction is preferably carried out with a low-fidelity polymerase, e.g. a Y-family polymerase such as DinB polymerase, Dpo4 polymerase, polymerase η, polymerase κ, polymerase I and polymerase v, a low-fidelity mutant of a low-fidelity polymerase, or a low-fidelity mutant of a high-fidelity polymerase such as those known in the art [5; 6]. At modified dC residues, a low-fidelity polymerase may introduce a nucleobase other than dG opposite to the modified methylated or non-methylated dC residue. For example, polymerase η is able to read through the modified dC residue, incorporating a dA upon reading past a hydroxylamine-modified dC residue.

As used herein, the term "fidelity" when used in reference to a polymerase is intended to refer to the accuracy of template-directed incorporation of complementary bases in a synthesized DNA strand relative to the template strand. Fidelity is measured based on the frequency of incorporation of incorrect bases in the newly synthesized nucleic acid strand. The incorporation of incorrect bases can result in point mutations, insertions or deletions. Fidelity can be calculated according to the procedures described in [7]. Methods for determining fidelity are well known to those skilled in the art.

As used herein, the term "high-fidelity" is intended to mean a frequency of accurate base incorporation that exceeds a predetermined value. Similarly, the term "low-fidelity" is intended to mean a frequency of accurate base incorporation that is lower than a predetermined value. The predetermined value can be, for example, a desired frequency of accurate base incorporation or the fidelity of a known polymerase.

As used herein, the term "polymerase mutant" is intended to refer to a polymerase having an amino acid sequence differing from the amino acid sequence of a selected wild-type polymerase in one or more amino acids. Preferably, the polymerase mutant comprises an amino acid sequence which is a least about 70%, more preferably at least about 80%, more preferably at least about 90%, more preferably at least about 95%, and most preferably at least about 99% identical to the amino acid sequence of the selected wild-type polymerase over its entire length.

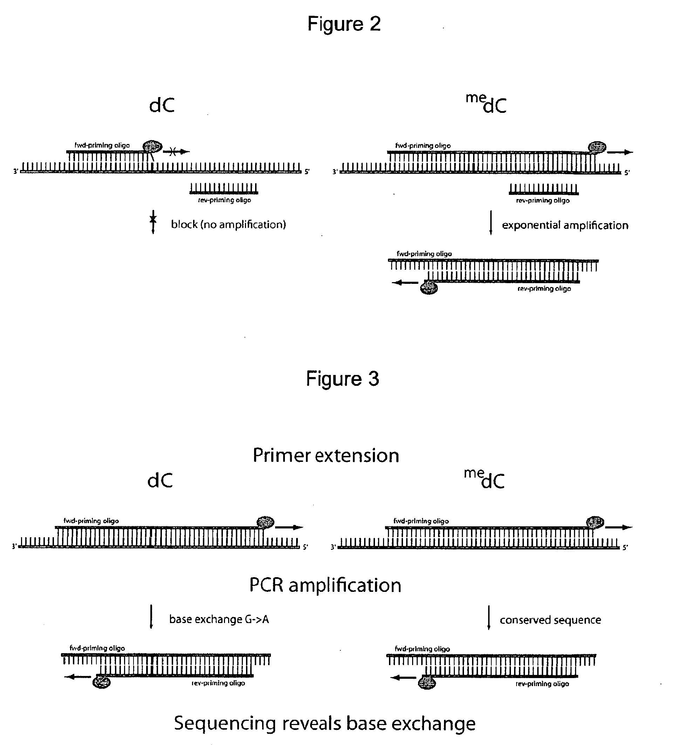

When the DNA polymerase reaction is carried out under conditions which cause blocking at defined modified bases resulting from the reaction of methylated or non-methylated cytosine residues, the sample may be subjected e.g. to quantitative PCR using a detectable label, e.g. a fluorescence label such as SYBR-Green as dsDNA-probe. The rationale behind this assay is that both chemical modifications represent an efficient block of polymerase extension. Therefore one has to expect strongly reduced amplification rates e.g. during Real-Time PCR for all modified cytosine or 5-methylcytosine residues. In the presence of unmodified nucleobases a normal qPCR signal is expected. By correlating the two applied reactions (e.g. Br⁺ and hydroxylamine treatment) with the control DNA, one can estimate differential methylation between samples. A schematic picture of a reaction block at reacted non-methylated cytosine residues is shown in Fig. 2.

When the DNA polymerase reaction is conducted under conditions which cause misreading at modified residues, a primer extension reaction may be carried out with a polymerase able to read through the lesions, causing incorporation of a base other than dG opposite the chemically modified cytosine or 5-methylcytosine base. In case of the hydroxylamine lesion, this is possible e.g. using Y-family error-prone polymerases such as polymerase η or Dpo4. Both preferentially insert an dA instead of dG opposite the modified site in a primer extension experiment, as determined by means of mass spectromery. Incorporation of dT and dC is negligible. The extended primer can be amplified by PCR. Detection of the misinsertion is performed by standard or modern sequencing methods, such as pyrosequencing. Here in the preferred case a dC/dT base exchange, which is similar to what is obtained in the bisulfite method, is detected. A schematic picture of this embodiment is shown in Fig. 3.

In order to provide adequate controls synthetic oligonucleotides with or without cytosine-methylations may be used as standards.

Further, the invention provides reagent kits suitable for carrying out the methods as described above. The reagent kits comprise reagents for introducing selective modifications at non-methylated or methylated cytosine residues and optionally further reagents for cleaving DNA strands at reacted positions and/or for carrying out a DNA polymerase reaction.

Further, the invention shall be explained in more detail by the following figures and examples.

### Brief description of the drawings

Fig. 1 shows a reaction scheme summarizing the analysis of DNA methylation patterns based on the bisulfite method.
Fig. 2 shows a schematic picture of a PCR reaction carried out under conditions, wherein polymerase extension is blocked at modified residues resulting from chemical modification of non-methylated cytosine or 5-methylcytosine.
Fig. 3 shows a schematic picture of a PCR reaction carried out under conditions, wherein misreading occurs at modified residues resulting from chemical modification of non-methylated cytosine or 5-methylcytosine.
Fig. 4 shows mechanisms and intermediates of the Vanadium (V) oxidation of ^{5Me}dC triggered in the absence and presence of LiBr.
Fig. 5 shows the sequence of oligonucleotide ODN(X) and a PAGE analysis of the strand cleavage after oxidation with a solution of 5 mM vanadium pentoxide and hot piperidine treatment.
Fig. 6 shows a PAGE analysis of (a) single-stranded oligonucleotides ODN(X) and (b) double-stranded oligonucleotides ODN(X)/ODN2 after treatment with a solution of 5 mM vanadium pentoxide, 10 mM LiBr in a 100 mM sodium phosphate buffer at 60°C for 18 h, followed by a 1 M piperidine treatment at 85°C for 20 min.
Fig. 7 shows the sequence and a PAGE analysis of oligonucleotide ODN(X,Y) after incubation in a solution of 1 mM NaIO₄, 4 mM LiBr, 50 mM H₂NCHO in 100 mM sodium phosphate buffer pH = 5 at 40°C during 1 h under ambient atmosphere and followed by a 1 M piperidine treatment at 85°C for 20 min.
Fig. 8 shows the difference in Ct values between non-methylated and methylated oligonucleotides ODN(Z) (ΔCt) at different concentrations. In ssDNA^{HA}, single-stranded oligonucleotides ODN(Z) were treated with hydroxylamine. In dsDNA^{HA}, the single-stranded oligonucleotides ODN(Z) were partially capped with complementary strands, leaving three bases in the middle of the oligonucleotide sequence single stranded, and were subsequently treated with hydroxylamine. In ssDNA, the single-stranded oligonucleotides ODN(Z) were not treated with hydroxylamine and used at the same concentration.
Fig. 9 shows the difference in Ct values between non-methylated and methylated oligonucleotides ODN(Z) (ΔCt) at different concentrations, with standard primers and hot-start primers (CleanAmp^{™} primers) of the same sequence having been used for amplification. HA: hydroxylamine-treated oligonucleotide; bg: untreated oligonucleotide.
Fig. 10 shows Real-time PCR data of oligonucleotides ODN(Z) at a template concentration of 20 fM. Non-templated reactions are suppressed.
Fig. 11 shows a Real-time PCR of oligonucleotides ODN(Z) at a template concentration of 200 fM, with the number of PCR cycles plotted against the dC/^{5Me}dC ratio. Oligonucleotides treated with hydroxylamine are represented by closed symbols, untreated controls by open symbols.
Fig. 12 shows a PAGE analysis of primer extension products obtained after hybridizing labelled primer 5'-Fluo-TACCACCTATCCTCT-3' to synthetic oligonucleotide 3'-ATGGTGGATAGGAGACAGT-5' and subsequently performing PCR using (a) different polymerases (Fig. 12A) and (b) varying concentrations of dA and dG (Fig. 12B).

### Example 1

### Selective detection of 5-methylcytosine residues in DNA

A selective oxidation of 5-methylcytosine residues in the presence of non-methylated cytosine and thymidine residues was carried out with vanadium (V) species or NaIO₄ in the presence of LiBr, whereby a conversion of the C5-C6 double bond of 5-methylcytosine was obtained.

### Material and Methods

**General.** Oligonucleotides ODN(X) and ODN2 were obtained from Metabion (Munich). Oligonucleotide ODN(X,Y) was obtained from IBA. The reagents and chemicals were purchased from Sigma, Aldrich, Across and TCI and were used without further purification. Na[VO₂(Hhida)].3H₂O was synthesized following procedures published elsewhere [8]. Na₆V₁₀O₂₈ was synthesized according to published procedures [9]. These two complexes were characterized using IR, ¹H and ⁵¹V NMR spectroscopy. For mass spectra a Bruker Flex III (for MALDI-ToF) was used. As matrix for DNA oligonucleotides a mixture of diammoniumcitrate, 15-crown-5 and 3'-hydroxy-picolinic acid in bidistilled water was used. If necessary, oligonucleotides were desalted using MF-Millipore membranes.

**Hybridisations.** All ODN:ODN hybridisations were performed in an Eppendorf Mastercycler Personal in buffer A heated up to 75°C and cooled within 3 h to 4°C.

**Vanadium treatment.** To 1 µL of ODN (10 µM), 2.5 µL of fresh V₂O₅ solution (20mM), 2 µL of LiBr (50 mM), 2 µL of Buffer A, B or C and 2.5 µL of autoclaved water were added. The resulting mixture was then vortexed, centrifuged and incubated at 60°C for 18 h. Following another centrifugation 100 µL of 0.3 M NaOAc and 1 mL of cold ethanol were added to the resulting mixture which was then placed at -80°C for 2 h. After centrifugation, the supernatant was removed and 10 µL of autoclaved water was added.

**Standard Periodate treatment.** To 1 µL of ODN (10 µM) was added 1 µL of NaIO₄ solution (20 mM), 1.6 µL of LiBr (50 mM), 2 µL of Buffer B and 4.4 µL of autoclaved water. The resulting mixture was then vortexed, centrifuged and incubated at 40°C for 1 h. Following another centrifugation, the solutions were desalted using MF-Millipore membranes.

**Piperidine Cleavage.** After vanadium or periodate treatment, to each sample, 100 µM of 1 M piperidine were added. The samples were heated to 80°C for 20 min, frozen, and lyophilized. Then, 10 µL water was added, and the samples were again lyophilized. The last step was repeated three times.

**PAGE Analysis.** Following the piperidine cleavage procedure 20µL bidistilled water and 20 µL buffer D were added to each sample. The separation was analyzed by standard polyacrylamide (20%) gel electrophoresis. The gel was then analyzed on a LAS3000 from Raytest.Only the fluorescein labelled strands appear as black bands.

**Buffers**

| Buffer | pH | NaH₂PO₄·H₂O (g/L) | Na₂HPO₄·7H₂O (g/L) |
|---|---|---|---|
| A | 3 | 68.995 | 0.016 |
| B | 5 | 68.07 | 1.80 |
| C | 7 | 29.18 | 77.33 |

| | | | |
|---|---|---|---|
| Buffer D = TBE loading buffer from Biorad. | | | |

### Results:

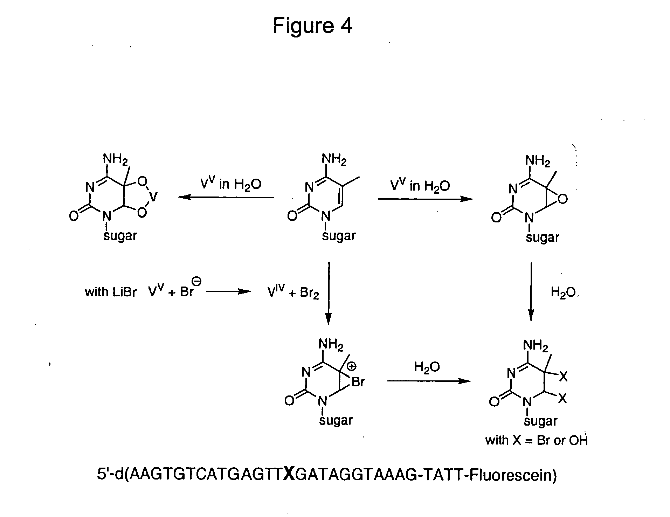

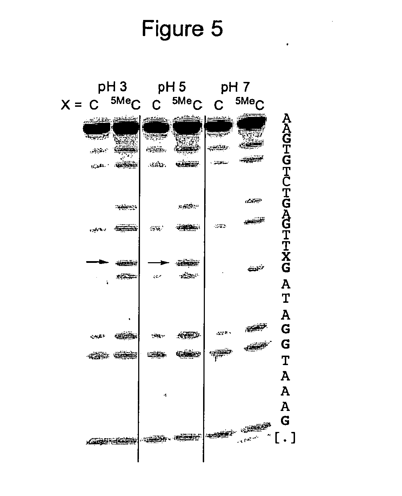

The reaction of V(V) compounds with the 5-methylcytosine residues is schematically depicted in Fig. 4. For the experiments, two 3'-fluorescein-labelled synthetic 30 base pair long single stranded oligonucleotides ODN(X) [5'-d(AAGTGTCATGAGTTXGATAGGTAAAGTATT-Fluo)-3'] were designed, which oligonucleotides contained either cytosine or 5-methylcytosine in the middle of the random DNA sequence (indicated by X). Both DNA strands were exposed to a series of V(V) containing clusters, having different redox properties. In order to further modulate the redox potential of the pyrimidine bases, the pH-value of the oxidation solution was varied. The obtained DNA products were subsequently treated with hot piperidine (85°C, 20 min) to induce strand breaks at those DNA bases, which were oxidatively damaged. The so treated DNA was finally analyzed by denaturing polyacrylamide gel electrophoresis, the result of which is shown in Fig. 5.

Freshly prepared V₂O₅ solutions showed an excellent selectivity for 5-methylcytosine oxidation at low pH values between 3 and 5. For the DNA strand with X = ^{5Me}dC, a clearly visible cleavage product was observed. No cleavage at this site occurred with X = dC, proving selective conversion of the C5-C6 double bond of ^{5Me}dC. No reaction at dT sites was detected, showing that oxidation reactions are indeed able to distinguish between all three pyrimidine bases dT, dC, and ^{5Me}dC in DNA. However, in both strands some cleavage at dG nucleobases was observed.

Addition of 10 mM LiBr to the reaction mixture immediately increased the selectivity for ^{5Me}dC reaction up to a factor of 4. In the presence of LiBr only the ODN with X = ^{5Me}dC furnishes a cleavage product at the expected ^{5Me}dC position after piperidine treatment.

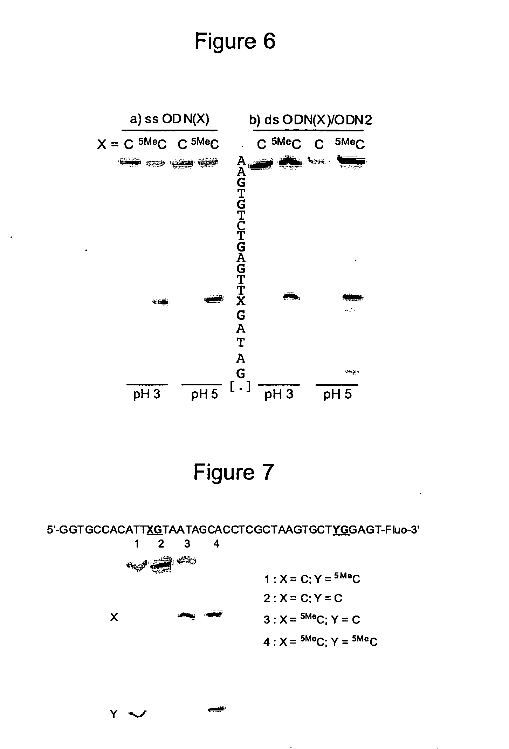

In order to further reduce the cleavage at dG sites we next performed the DNA oxidation in the absence of oxygen. As depicted in Fig. 6a, reaction of DNA with V₂O₅ in the presence of LiBr and in the absence of oxygen allowed a highly specific reaction at the C5-C6 double bond of the ^{5Me}dC base. No reaction at any other nucleobase was then observable. Neither cleavage at dT nor at purine positions was detected, even at the rather high temperature of 80°C. The requirement of high temperature enabled us to perform the analysis even directly at double strands as these melt to a significant extend at these high temperatures. The counter strand (ODN2) 5'-d (AATACTTTACCTAT^{5Me}CGAACTCATG-ACACTT)-3' was hybridised with each ODN(X). Treatment of the corresponding double strand under the same oxidation conditions allowed selective cleavage at the ^{5Me}dC position as depicted in Fig. 6b.

In the following, a number of further oxidizing agents and halogenide sources were scanned. The replacement of the lithium salts by sodium or potassium salts had no effect. Bromides gave better results than other halogenides. Sodium tungstate showed an analogous reactivity compared to V₂O₅ but provided also higher amounts of dG oxidation. The best results were obtained with iodine containing oxidizing reagents such as ortho-iodoxybenzoic acid (IBX), Dess-Martin periodinane (DMP) or sodium periodate. The three oxidants gave excellent conversion of ^{5Me}dC without any detectable dG oxidation and this even in the presence of oxygen. The best results were finally obtained with a combination of NaIO₄/LiBr (1 mM NaIO₄, 4 mM LiBr, 100 mM Na₃PO₄ buffer pH = 5, 40°C, 1 h). This system allowed selective conversion of the C5-C6 double bond of ^{5Me}dC, at a strongly reduced reaction temperature of just 40°C. In addition, the reaction could be performed even in the presence of oxygen.

Further, the ability to detect ^{5Me}dC in real d(CpG) sites was investigated, which are the epigenetic control elements. The p16 gene is described as a regulator of the invasive proliferation of basal cell carcinoma cells. The regulatory function of the promoter of this gene is reduced by a diminished methylation status at certain d(CpG) sites [10]. A short 41 base pair long fragment of the p16 promoter (residues 1751-1791; 5'-GGTGCCACATTXGTAATAGCACCTCGCTAAGTGCTYGGAGT-Fluo-3') containing two of the critical d(CpG) sequences (indicated as X and Y) was selected.

With X and Y either dC or ^{5Me}dC, four different methylation patterns are possible in this oligonucleotide. Treatment of ODN(X,Y) under the described conditions provided indeed selective reaction at the ^{5Me}dC sites, as shown by PAGE analysis. In addition, however, some reaction at dT sites was observed. This problem could be efficiently overcome by the addition of formamide (50mM) to the oxidizing solution. Fig. 7 depicts the results of these studies showing extremely efficient detection of ^{5Me}dC nucleobases in mixed DNA sequences. Note that there are nearly no side reactions occurring at other nucleobases.

In conclusion, we developed a novel chemical method which allows to target ^{5Me}dC in DNA. Selective detection of ^{5Me}dC in the presence of all other canonical nucleobases dC, dT, dA and dG was achieved using either a combination of V₂O₅/LiBr or NaIO₂/LiBr. We believe that this chemistry proceed through the formation of a bromonium cation intermediates on the C5-C6 double bond of pyrimidines. The sensitivity of the method may be increased e.g. by linking the method either to the PCR reaction or to the silver staining technology described in [11]. This should establish a rapid and easy to use analysis of epigenetic information in genes from biological samples.

### Example 2

### Selective detection of non-methylated cytosine residues in DNA

A selective modification of non-methylated cytosine residues in defined DNA samples was carried out using hyroxylamine.

### Material and Methods

General. Synthetic oligonucleotides ODN(Z), capping oligonucleotides and primers were purchased from Thermo Hybaid (Ulm). Vent exo- DNA polymerase was obtained from New England Biolabs. The reagents and chemicals were purchased from Sigma-Aldrich and New England Biolabs and were used without further purification.

**Protection of oligonucleotides.** Double-strand protection of oligonucleotide ODN(Z) was achieved by mixing equimolar amounts of template ODN(Z) [5'-(CGGGGAGCAGCATGGAGCCTTZGGCTGACTGGCTGGCCACGGC)-3'], capping oligonucleotide #1 [5'-(GCCGTGGCCAGCCAGTCAGC)-3'] and capping oligonucleotide #2 [5'-(AGGCTCCATGCTGCTCCCCG)-3'], heating to 95°C and cooling slowly to 4°C.

**Hydroxylamine treatment.** 7 µl of double-strand protected ODN(Z) as obtained above were treated with 20 µl of 4 M hydroxylamine hydrochloride, pH 6 adjusted with diethylamine, at 20°C for 14 h. Then, 25 µl of 3 M NaOAc were added and filled with water to 250 µl. The DNA was precipitated by the addition of 1250 µl of 2-propanol for 2 h at -80°C. This procedure was repeated once and the final pellet was resuspended in 10 mM Tris/Cl, pH 8. The amount of DNA was quantified spectroscopically. For PCR quantification, a dilution series from 20 pM to 0.2 pM was prepared.

**Real time PCR.** Quantitative real-time PCR was performed on a realplex thermal cycler (Eppendorf) using SYBR-green as dsDNA probe. The 20 µl reactions contained 200 µM dNTP, 1 U Vent exo- DNA polymerase, 500 nM of each of primers 5'-(GCCGTGGCCAGCCAGTCAGC)-3' and 5'-(CGGGGAGCAGCATGGAGCCT)-3', and 2 mM MgSO₄ (ThermoPol buffer). The PCR program used was as follows: 2 min at 95°C, 40 x (15 s at 95°C/10 s at 63°C/10 s at 72°C).

In a second experiment, hot-start versions of the above primers containing a heat-labile block at the 3'-end were used. These CleanAmp^{™} precision primers were obtained from TriLink Biotechnologies (San Diego).

### Results

In order to show specific detection of non-methylated dC residues in a DNA sample, two 43 base pair long single-stranded oligonucleotides ODN(Z) representing a fragment of the p16 promoter (residues 21964813-21964771 of Hs9_8570; NT 008413.17) were designed, which oligonucleotides contained either cytosine or 5-methylcytosine in the middle of their sequence (indicated by Z). These single-stranded oligonucleotides ODN(Z) were subjected to hybridization with capping oligonucleotides #1 and #2, leaving a short stretch of DNA single-stranded. Subsequently, the exposed residue was treated with hydroxylamine to selectively modify non-methylated cytosine residues. After purification, the treated material was used as a template in a PCR reaction with primers surrounding the residue in question.

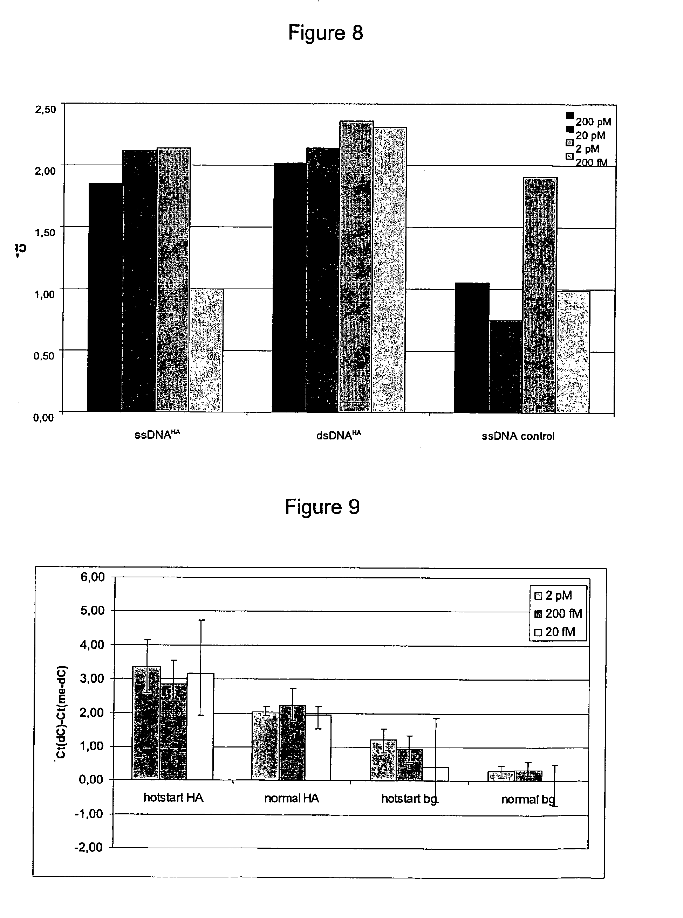

Subsequently, a dilution series of hydroxylamine-treated DNA (200 pM, 20 pM, 2 pM and 200 fM) was subjected to real-time analysis. All samples were run as triplicates and their mean Ct values were calculated using the the realplex 1.5 software (Eppendorf). The results are shown in Table 1 and Fig. 8.

**Table 1**

| | 200 pM | Dev | ΔCt | 20 pM | Dev | ΔCt | 2 pM | Dev | ΔCt | 200 fM | Dev | ΔCt |
|---|---|---|---|---|---|---|---|---|---|---|---|---|
| ssDNA^{HA}-C | 12,09 | 0,06 | 1,85 | 15,4 | 0,31 | 2,12 | 18,67 | 0,15 | 2,14 | 21,03 | 0,13 | 1 |
| ssDNA^{HA}- meC | 10,24 | 0,31 | | 13,28 | 0,06 | | 16,53 | 0,15 | | 20,03 | 0,08 | |
| dsDNA^{HA}-C | 10,79 | 0,29 | 2,02 | 14,17 | 0,44 | 2,14 | 17,74 | 0,15 | 2,36 | 20,6 | 0,38 | 2,13 |
| dsDNAmeC | 8,77 | 0,07 | | 12,03 | 0,15 | | 15,38 | 0,05 | | 18,47 | 0.04 | |
| ssDNA C | 8,9 | 1,05 | 1,05 | 11,8 | 0,92 | 0,75 | 16,25 | 0,09 | 1,91 | 18,52 | 0,15 | 0,99 |
| ssDNA meC | 7,85 | 0,2 | | 11,05 | 0,16 | | 14,34 | 0,14 | | 17,53 | 0,13 | |
| no template | 21,38 | 0,13 | | | | | | | | | | |

Fig. 8 shows that double-stranded (capped) templates yield the most reliable results, even down to 200 fM concentration. The detection limit is conditional upon the Ct value of a non-templated control reaction employing only buffer, primers, dNTP and polymerase (cf. Table 1: no template). This limit has been reached e.g. at 200 fM for the single-stranded template, explaining the reduced ΔCt value of 1.

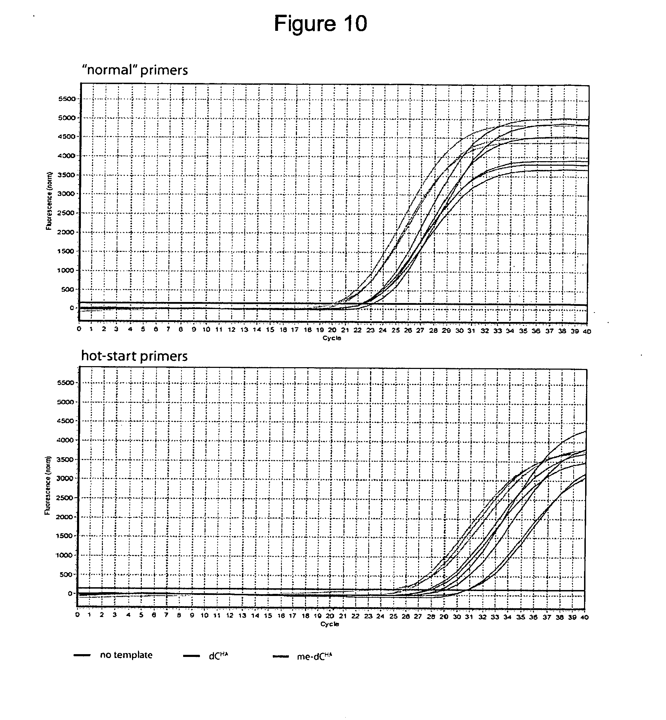

In order to suppress unspecific amplification of primer dimers and to increase the detection limit, hot-start conditions may be used. As can be derived from Fig. 10, the use of hot-start primers shifts the detection limit to lower concentrations, allowing template concentrations below 20 fM. The phenomenon of elevated Ct differences observed for the untreated control samples has to be further evaluated. However, it appears that 5-methylcytosine itself may result in accelerated amplification rates, even without hydroxylamine treatment. Differential incorporation of nucleobases opposite methylated residues has been described previously [12] and does not interfere with the proposed procedure. The observed effect is further amplified by the hydroxylamine treatment.

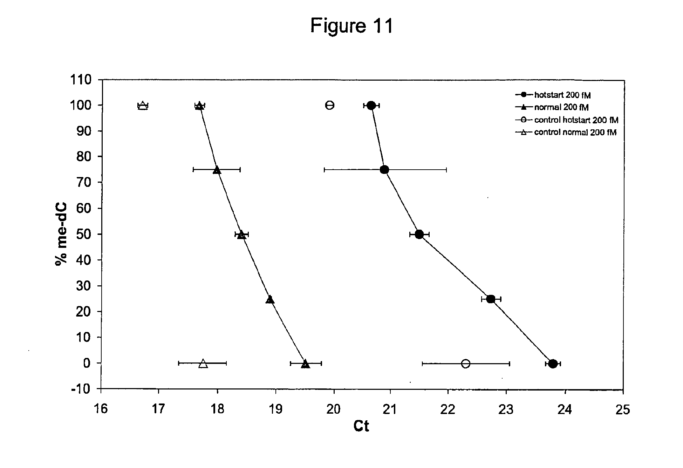

For evaluating the linearity of the system for quantification, a standard curve of various dC/^{5Me}dC ratios was prepared at a template concentration of 200 fM. The PCR conditions were as described above, except that 5 min instead of 2 min initial denaturation was employed to allow more hot-start primers to become deprotected. Fig. 11 shows that the linearity of ^{5Me}dC quantification is good at concentrations below 50%. A decrease in linearity at concentrations above 50% was expected due to the low amount of attackable sites. This region is covered by the ^{5Me}dC specific oxidative treatment.

To further suppress background amplification (here: bypass of damaged dC residues), a piperidine cleavage reaction may be performed prior to the Real-time PCR reaction.

In order to detect modified dC or ^{5me}dC residues via misincorporation of nucleobases, synthetic oligonucleotide 3'-ATGGTGGATAGGAGACAGT-5' was treated with hydroxylamine and hybridized with a primer ending at the fourth last base (C) of the template. Primer extension was performed by providing all four nucleotides and varying (a) the polymerase and (b) the concentration of either dG or dA.

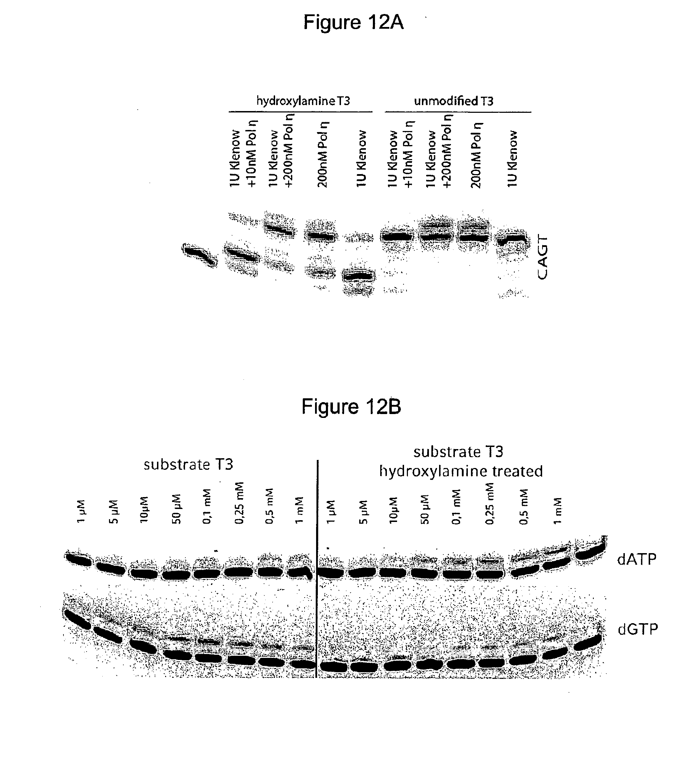

As can be seen from Fig. 12A, full primer extension was only achieved by means of polymerase η and on the untreated control strand. On the other hand, Fig. 12B shows preferential incorporation of dA into the primer after treating the template with hydroxylamine, whereas dG is incorporated in only minor amount. These data strongly suggests that low-fidelity polymerases such as polymerase η utilize dA instead of dG in the absence of templating information by the hydroxylamine-modified nucleobase.

### Example 3

### Quantification of methyltransferase activity

For quantifying methyltransferase activity, the previously deployed 43 base pair long fragment of the p16 promoter [5'-(CGGGGAGCAGCATGGAGCCTTCGGCTGACTGGCTGGCCACGGC)-3'] was hybridized with its complementary sequence [3'-(GCCCCTCGTCGTACCTCGGAAG^{5Me}CCGACTGACCGACCGGTGCCG)-5'] methylated at the indicated position to provide a double stranded hemimethylated construct. The unmethylated strand may be biotinylated to facilitate subsequent purification.

The double stranded hemimethylated construct serves as a substrate of Dnmt1 together with S-adenosyl-methionine (SAM) as second substrate e.g. in IC₅₀ determinations or simple activity assays, either with purified methyltransferases or extracts from inhibitor-treated material. Subsequently, this DNA substrate is purified by standard methods (e.g. phenol/chloroform extraction, precipitation or streptavidine purification) and the formerly non-methylated strand of the p16 promoter is hybridized to an excess of capping oligonucleotides, as described in Example 2. This results in a partially single-stranded construct that gives access to hydroxylamine in the subsequent treatment. Detection proceeds as described previously. In parallel, a standard curve of defined dC/^{5Me}dC ratios with identical oligonucleotides is recorded by the same method. The IC₅₀ value is determined by the concentration of inhibitor that resulted in a 50% decrease of methylation under the respective assay conditions.

### Reference List

[1] M. Esteller, Nat. Rev. Genet. 2007, 8, 286-298.
[2] A.M. Maxam, W. Gilbert, Proc. Natl. Cad. Sci. USA 1977, 74, 560-564.
[3] C.M. Rubin, C.W. Schmid, Nucl. Acids Res. 1980, 8, 4613-4619.
[4] a) E. Frese, E. Bautz, E. Bautz Freese, Proc. Natl. Acad. Sci. USA 1961, 47, 845-855; b) D.M. Brown, P. Schell, J. Chem. Soc. 1965, 208-215.
[5] B.D. Biles, B.A. Connolly, Nucl. Acids Res. 2004, 32, e176.
[6] M. Suzuki, A.K. Avicola, L. Hood, L.A. Loeb , J. Biol. Chem. 1997, 272, 11228-11235.
[7] K.R. Tindall, T.A. Kunkel, Biochemistry 1988, 27, 6008-6013.
[8] M. Mahroof-Tahir, A.D. Keramidas, R.B. Goldfarb, O.P. Anderson, M.M. Miller, D.C. Crans, Inorg. Chem. 1997, 36, 1657-1668.
[9] R. Ksiksi, M. Graia, T. Jouini, Acta Cryst., 2005, E61, il177-il179
[10] S.S. Månsson, J. Reis-Filho, G. Landberg, J. Pathol., 2007, ASAP*.*
[11] a) G.A. Burley, J. Gierlich, M.R. Mofid, H. Nir, S. Tal, Y. Eichen, T. Carell, Thomas, J. Am. Chem. Soc. 2006, 128, 1398-1399; b) J. Gierlich, G.A. Burley, P.M.E. Gramlich, D.M. Hammond, T. Carell, Org. Lett. 2006, 8, 3639-3642.
[12] A. Bart, M.W.J. van Passel, K. van Amsterdam, A. van der Ende, Nucl. Acids Res. 2005, 33, e124.

## Claims

1. A method for determining methylation at cytosine residues in DNA, comprising the steps:
(a) providing a sample comprising a single-stranded DNA segment comprising at least one cytosine residue,
(b) treating a fraction of the sample from step (a) with a first reagent which selectively reacts with 5-methylcytosine residues in said single-stranded DNA segment to obtain modified residues,
(c) treating a fraction of the sample from step (a) with a second reagent which selectively reacts with non-methylated cytosine residues in said single-stranded DNA segment to obtain modified residues, and
(d) detecting said modified residues.

2. The method of claim 1 wherein said single-stranded DNA segment comprises at least one d(CpG) dinucleotide.

3. The method of claim 1 or 2 wherein said single-stranded DNA segment is flanked by at least one non-single stranded segment.

4. The method of claim 3 wherein said non-single stranded segment is a double- or triple-stranded DNA segment.

5. The method of any one of claims 1 to 4 wherein step (b) comprises reacting said 5-methylcytosine residues with an electrophilic/oxidizing species under conditions wherein non-methylated cytosine residues are substantially inert.

6. The method of claim 5 wherein the electrophilic species is generated by oxidizing a precursor with an oxidant.

7. The method of claim 6 wherein the oxidant is an iodine-containing compound such as ortho-iodoxybenzoic acid, Dess-Martin periodinane and a periodate, a permanganate, a perruthenate, a vanadium (V)-containing compound, a molybdenum (VI)-containing compound, a tungsten (VI)-containing compound or a peroxide.

8. The method of any one of claims 5 to 7 wherein the electrophilic species is Br⁺, Cl⁺, I⁺, NO⁺, NO₂⁺, SO₃H⁺, a carbon-based electrophilic species or an electrophilic oxygen species, particularly Br⁺.

9. The method of any of claims 1 to 8 wherein step (c) comprises reacting said non-methylated cytosine residues with a nucleophilic reagent under conditions wherein 5-methyl dC residues are substantially inert.

10. The method of claim 9 wherein said nucleophilic reagent is hydroxylamine, an N- or O-substituted hydroxylamine, or a substituted hydrazine.

11. The method of any one of claims 1-10 wherein the detection comprises subjecting said treated sample fractions from step (b) and/or (c) to a chemical treatment, e.g. a base treatment, whereby a DNA strand cleavage occurs at positions of modified residues.

12. The method of any one of claims 1-10 wherein the detection comprises subjecting said treated sample fractions from step (b) and/or (c) to a polymerase reaction under conditions whereby blocking and/or misreading of the DNA polymerase reaction occurs at positions of modified residues.

13. The method of claim 12 wherein the polymerase reaction is carried out using hot-start conditions.

14. The method of claim 12 or 13 wherein the polymerase reaction is carried out under conditions where blocking at modified residues occurs.

15. The method of claim 14 wherein the polymerase reaction is carried out with a high-fidelity DNA polymerase, e.g. Taq DNA polymerase, Pfu DNA polymerase, Vent DNA polymerase, Vent exo- DNA polymerase, KOD DNA polymerase and BstPoll DNA polymerase, or a high-fidelity mutant of a high-fidelity DNA polymerase.

16. The method of claim 12 or 13 wherein the polymerase reaction is carried out under conditions where misreading at modified residues occurs.

17. The method of claim 16 wherein the polymerase reaction is carried out with a low-fidelity DNA polymerase, e.g. an Y-family polymerase such as DinB polymerase, Dpo4 polymerase, polymerase η, polymerase κ, polymerase and polymerase v, a low-fidelity mutant of a low-fidelity DNA polymerase or a low-fidelity mutant of a high-fidelity DNA polymerase.

18. A method for determining 5-methylcytosine residues in DNA, comprising the steps:
(a) providing a sample comprising a single-stranded DNA segment comprising at least one cytosine residue,
(b) treating a fraction of the sample from step (a) with a reagent which selectively reacts with 5-methylcytosine residues in said single-stranded DNA segment to obtain modified residues, and
(c) detecting said modified residues.

19. A method for determining non-methylated cytosine residues in DNA, comprising the steps:
(a) providing a sample comprising a single-stranded DNA segment comprising at least one cytosine residue,
(b) treating a fraction of the sample from step (a) with a reagent which selectively reacts with non-methylated cytosine residues in said single-stranded DNA segment to obtain modified residues, and
(c) detecting said modified residues.

20. The method of any of claims 1 to 19 for the analysis of DNA methylation patterns in epigenetics.

21. The method of any of claims 1 to 19 for the quantification of methyltransferase activity.

22. A reagent kit for determining methylation at cytosine residues in DNA, comprising:
(a) a first reagent which selectively reacts with 5-methylcytosine residues in a single-stranded DNA segment to obtain modified residues,
(b) a second reagent which selectively reacts with non-methylated cytosine residues in a single-stranded DNA segment to obtain modified residues, and
(c) optionally further reagents for carrying out a chemical cleavage of DNA at modified residues and/or reagents for carrying out a DNA polymerase reaction.

23. Use of the reagent kit of claim 22 in a method of any one of claims 1 to 19.
